(19) Europäisches Patentamt

European Patent Office

Office européen des brevets

(11) EP 1 245 203 B1

(12) EUROPEAN PATENT SPECIFICATION

(45) Date of publication and mention
of the grant of the patent:
**08.03.2006 Bulletin 2006/10**

(51) Int Cl.:
*A61F 2/06* (2006.01)

(21) Application number: **02007150.2**

(22) Date of filing: **28.03.2002**

(54) **Stent**

Stent

Stent

(84) Designated Contracting States:
**AT BE CH CY DE DK ES FI FR GB GR IE IT LI LU MC NL PT SE TR**

(30) Priority: **30.03.2001 JP 2001101683**

(43) Date of publication of application:
**02.10.2002 Bulletin 2002/40**

(73) Proprietor: **TERUMO KABUSHIKI KAISHA Tokyo (JP)**

(72) Inventor: **Ishii, Tatsuzo**
**Ashigarakami-gun,**
**Kanagawa (JP)**

(74) Representative: **TBK-Patent**
**Bavariaring 4-6**
**80336 München (DE)**

(56) References cited:
**WO-A-01/01889          WO-A-97/25937**
**WO-A-98/30173          US-A- 5 913 897**
**US-B1- 6 200 337**

**Description**

The present invention relates to a stent, which is used in the body duct (passage way), for expanding an angiostenotic portion, closing an aneurysmal opening, securing the urethra due to urethral dilation of a prostatomegaly portion, etc. More specifically, the present invention relates to a flexible stent which is inserted in a curved duct in the interior of the body to form a smooth spiral around an axial direction in which the stent is easily placed, and continuous protruding portions on the spiral are arranged on the curved surface of a cylindrical wall portion, so that the entire protruding portions are formed an arcuate structure constituting a recessed surface in an oblique direction inward with respect to the stent, which is relatively free from being caught during operation in the body duct.

Description of the Related Art:

A catheter equipped with a balloon whose size at the time of swelling is previously determined is used for angioplasty and treatment of other body ducts or treatment of defective vessel portions. A stent is arranged on the cylindrical portion of the folded balloon so as to cover the portion, and the catheter is inserted into the body, with the balloon being positioned at the portion to be treated; by supplying fluid to this balloon, the balloon is dilated, and the stent is dilated by this dilatable force. However, when the stent is not easily bent in the axial direction, it is rather difficult for the stent to pass through a curved body duct. Fig. 7 shows the stent as disclosed in JP 2-174859 A (USP 5195984 B) in which thin and narrow stainless steel members are arranged in the axial and circumferential directions of the stent to form an annular member, and such annular members are arranged in the axial direction of the stent, forming a pattern in which the protrusions of adjacent annular members are connected by connector members parallel to the axial direction, so that if the stent is bent, it is deformed like a hinge at the portion where the annular members are connected, with the protrusions of the annular members protruding and likely to get caught in the curved body duct. Further, since the protruding portion of this protrusion is longitudinally linear, it is outwardly deformed like the tip of a tulip petal when a force in a perpendicular direction is applied, the portion likely to be warped outwardly and likely to get caught.

Fig. 8 shows the stent as disclosed in JP 2000-5101328 A (WO 98/14137), in which a stent as shown in Fig. 6 is formed by bending a wire into a corrugated strip, which is spirally wound around into a cylinder, with the ends of the spiral member being fixed to the ends of a meandering wire parallel to the axial direction of the stent. In this stent, the surfaces of the protrusions of the corrugated portion are tangentially in contact with the cylinder, so that the stent protrudes tangentially from the cylinder. Further, the axially extending wire for fixing the ends of the spiral member involves a portion where wires are superimposed one upon the other, and this superimposed portion has a thickness double the wire diameter, and further causes the corrugated portion to protrude outwardly. If this stent is inserted into a body duct having many twists and turns like a vessel, the corrugated portion is likely to get caught by the inner wall of the body duct.

Further, WO 01/01889 A1 discloses a stent according to the preamble of claim 1.

SUMMARY OF THE INVENTION

It is an object of the present invention to provide a stent which can be smoothly inserted into a curved body duct and which does not easily get caught in the body duct and can be easily placed in the body duct.

According to the present invention, there is provided a stent as set forth in claim 1.

Also, as to a stent described above, the protruding portions of a first ribbon-like body and the non-protruding portions of a second ribbon-like body adjacent to the first ribbon-like body get into each other in the direction of the central lines of the protruding portions of the first ribbon-like body to thereby form a cylindrical wall portion with high density with the thin and narrow members.

As to a stent described above, the protruding portions of the thin and narrow member form the respective ribbon-like body in a continuous wave-like or continuous zigzag-shaped structure.

As to a stent described above, each protruding portion of the thin and narrow member constitutes a part of a rhombus or ellipsoid, adjacent rhombi or ellipsoids being connected with each other by a thin and narrow member on its minor or major axis to form the respective ribbon-like body as a whole in the form of a rosary.

As to a stent described above, the connecting members are provided at intervals of 60 to 180 degrees with respect to one round of 360 degrees along the longitudinal direction of the spiral of the respective ribbon-like body.

As to a stent described above, the cylindrical wall portion consists of a metal or plastic cylindrical wall portion with a plurality of openings formed in the wall surface thereof.

As to a stent described above, the central lines of the protruding portions of the ribbon-like bodies are at an angle of 20 to 70 degrees with respect to the major axis line parallel to the central axis of the cylindrical body and passing the surface of the wall portion.

The present invention will be described hereinafter in more detail with reference to the following drawings, however the invention in not limited these embodiments of the drawings.

BRIEF DESCRIPTION OF THE DRAWINGS

Fig. 1 is a development showing an embodiment of the present invention;
Fig. 2 is a development showing the structure of protruding portions in the another embodiment of the present invention;
Fig. 3 is a development showing another embodiment of the present invention;
Fig. 4 is a partial development showing another connecting member of the embodiment of the present invention;
Fig. 5 is a development showing another embodiment of the present invention;
Fig. 6 is a partial development showing another embodiment of the present invention;
Fig. 7 is a perspective view showing a conventional stent; and

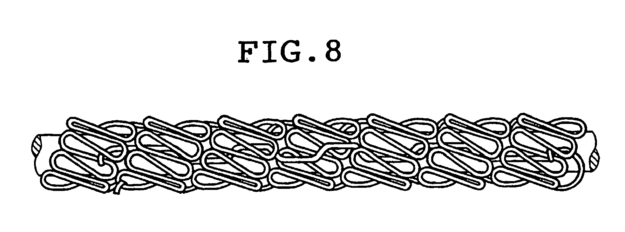

Fig. 8 is a perspective view showing a conventional stent.

DESCRIPTION OF THE PREFERRED EMBODIMENTS

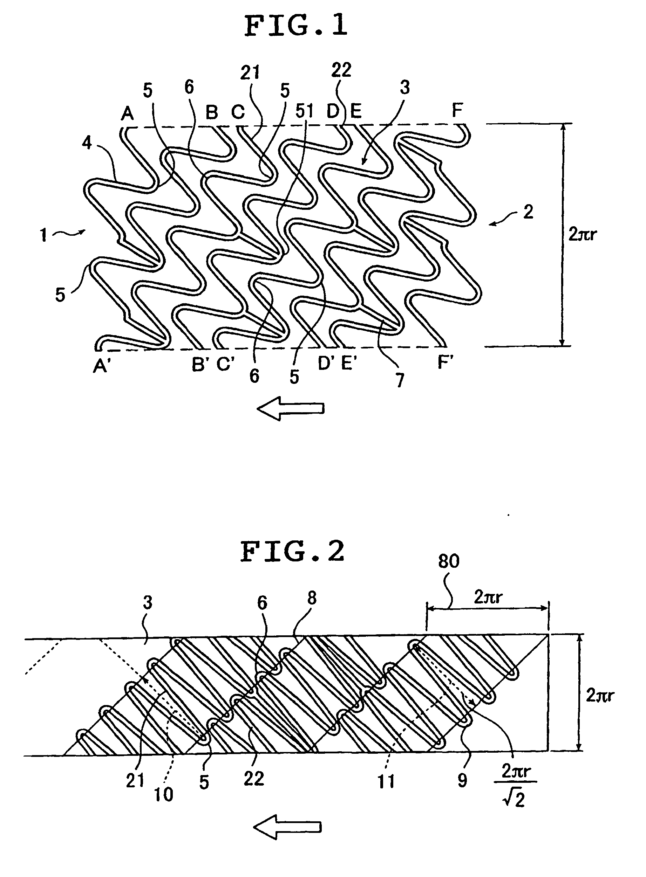

Fig. 1 is a development showing a stent according to a preferred embodiment of the present invention. In the development, the vertical axis indicates the circumference of a cylindrical wall portion. Assuming that the radius of the cylindrical member is r, the circumference is 2πr. The horizontal axis indicates the major axis line (longitudinal direction) of the cylindrical wall portion. The arrow indicates the direction in which the stent is inserted into a body duct.

The stent of the present invention is in the whole formed by a cylindrical wall portion 3 having a distal end 1 and a proximal end 2, the stent pattern forming the curved surface of the cylindrical wall portion.

The pattern is formed by thin and narrow members 4. The cross-section of the thin and narrow members 4 should be such that the bottom surface thereof facing the central axis of the stent constitutes a curve having a curvature in conformity with the curved surface of the cylindrical wall portion; as for the other portion, it is possible to use linear members having various general sectional structures, such as rectangular, square, beveled or rounded, elliptical, circular, or modified shape.

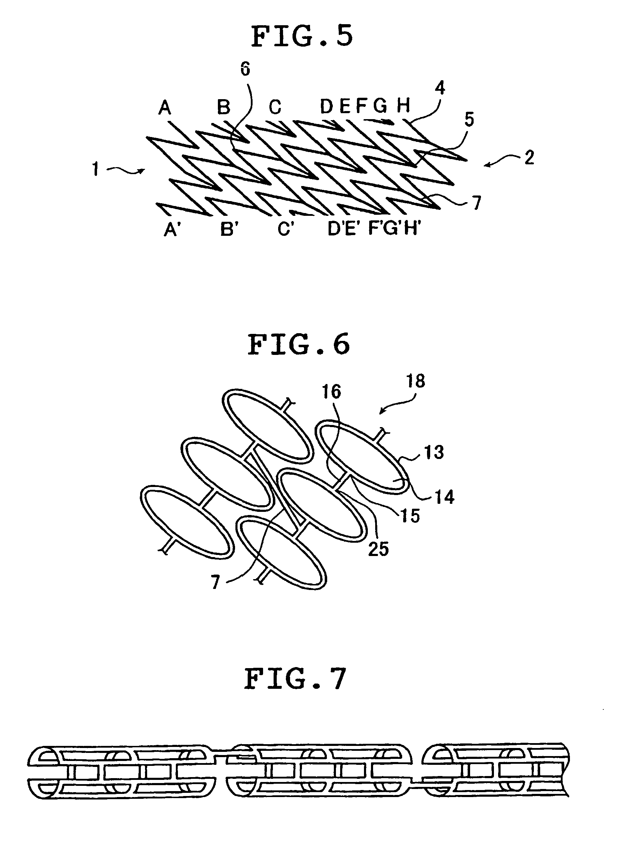

The pattern formed by thin and narrow members 4 is characterized in that it has portions 5 where the thin and narrow members protrude and portions 6 where they do not protrude. The protruding portions 5 and the non-protruding portions 6 may be wave-shaped as shown in Fig. 1, zigzag-shaped as shown in Fig. 5, elliptical as shown in Fig. 6, rhombic (not shown), or of modified shape. It is possible for the protruding portions 5 and the non-protruding portions 6 to be connected together at random. However, it is more desirable for them to be continuously connected in substantially the same structure to form a pattern, for that will enable the vessel wall or-the like to be uniformly supported when the stent is dilated.

As shown in the development of Fig. 1, connection is established between portions A and A', portions B and B', portions C and C', portions D and D', portions E and E', and portions F and F'. While Fig. 1 shows a pattern in which two kinds of ribbon-like bodies are arranged side by side and wound spirally, this should not be construed restrictively. It is also possible to adopt a pattern in which three kinds of ribbon-like bodies are arranged side by side and wound spirally, or a pattern in which one kind of ribbon-like body is wound spirally. In this case, it is possible to adopt a structure in which, as described below, adjacent ribbon-like bodies get into each other; by performing winding with the spiral laid, it is possible to form a pattern in which the ribbon-like bodies are superimposed one upon the other without changing the amplitudes of the protruding portions 5 and the non-protruding portions 6.

Fig. 2 is a development showing a stent according to an another preferred embodiment of the present invention. While Fig. 2 shows a pattern in which one kind of ribbon-like body is arranged side by side and wound spirally. In this example, the protruding portions 5 of a ribbon-like body 21 and the non-protruding portions 6 of an adjacent ribbon-like body 22 come near and put into each other in the direction of the central lines 10 of the protruding portions 5, so that the thin and narrow members can form a wall portion with high density, and the vessel wall or the like can be sufficiently supported if the stent is expanded. In the case of a pattern in which two or three kinds of ribbon-like bodies are spirally wound, it is possible for the distance through which they come near and put into each other to be large, thereby making it possible to increase the density of the thin and narrow members on the cylindrical wall portion.

Here, the wave-like protruding portions 5 of the thin and narrow members are provided such that their central lines 10 indicated by the dotted lines in Fig. 2 are at an angle with respect to a major axis line parallel to the central axis of the cylindrical portion and passing the surface of the wall portion. In the conventional stent shown in Fig. 7, the protrusions are in the same direction as the central axis of the cylindrical body, so that when the entire stent is bent along the blood vessel or the like, the protrusions will stick out to get caught in the body duct. The stent of the present invention is free from such a problem. As described below, the protrusions 5 of the stent of the present invention forms in the whole an arcuate body along the curved surface of the cylindrical wall portion, which further helps to solve the above problem.

To form the entire protruding portions into an arcuate structure, it is desirable for the central lines 10 of the wave-like protruding portions 5 of the thin and narrow members to be at an angle of 20 to 70 degrees, and more preferably, 30 to 60 degrees, with respect to the major axis line on the cylindrical wall portion parallel to the central axis of the cylindrical body. With this structure, the protruding portions 5 of the thin and narrow members form an arcuate body along the curvature of the circumference of the cylindrical wall portion.

This will be illustrated with reference to the development of Fig. 2. In Fig. 2, the longitudinal direction of the spiral is indicated by thin lines 8 extending obliquely upward to the right at an angle of 45 degrees; when one ribbon-like body intersects the central axis of the cylindrical body at 45 degrees, the spiral pitch is $2\pi r$ on the major axis line of the cylindrical wall portion so that the wave-like protruding portions 9 in the whole are at an angle of 90 degrees with respect to the thin lines 8. In this case, the spiral pitch 80 on the major axis line of the cylindrical wall portion is $2\pi r/\sqrt{2}$. Here, the circumference of the ellipsoid obtained by cutting the cylinder at an angle of 45 degrees is as follows.

In a cylindrical body with a radius r, the major axis radius $a = \sqrt{2}r$, and the minor axis radius b = r. Thus,

$$P = \pi \sqrt{2(a^2 + b^2) - (a - b)^2} \, / \, 2.2 = 1.225 \times 2\pi r$$

The ratio of the spiral width to the circumference of the ellipsoid is approximately 0.58. Thus, the wave-like protruding portions form an arcuate body along the cylindrical wall portion and occupy not less than 0.58 times the circumferential length.

When the pattern shown in Fig. 2 is formed by one kind of ribbon-like body, with portions of the ribbon-like body being superimposed one upon the other, the length of the wave-like protruding portions 9 is longer than the spiral width 11, as shown in the drawing, so that the wave-like protruding portions 9 hold the cylindrical body in such a manner as to hold an ellipse like a crab holding a thing with its arms. Each of the wave-like protruding portions assumes this form, whereby the stent is prevented from detaching from the balloon, and since the wave-like protruding portions are wound around into the balloon, the stent rarely gets caught in the body duct when inserting it into a meandering vessel.

Further, the wave-like protruding portions may also form a short pattern in which their length is less than half the circumference of the ellipsoid in section obtained by cutting the cylindrical body in the direction of the center line 10 indicated by the dotted line. In this case also, the whole assumes an arcuate structure, so that warpage in the opposite direction does not easily occur, and the stent is obviously free from getting caught in the body duct when it is inserted into a meandering vessel.

Further, the connecting members 7 connecting the adjacent ribbon-like thin and narrow members, which are formed of thin and narrow members, are at an angle with respect to the line 8 indicating the spiral pattern of the ribbon-like bodies. Further, the connecting members 7 are connected such that before and after deformation of the thin and narrow members caused by expansion and contraction of the stent in diameter, the distance of the central line 10 of the ribbon-like bodies is substantially the same. Further, while at least one connecting member 7 is provided for one round (360 degrees) of the ribbon-like body around the cylindrical body, it is desirable, from the viewpoint of securing the flexibility of the stent, that at least two of the wave-like protrusions of the ribbon-like body are not connected by the connecting members 7.

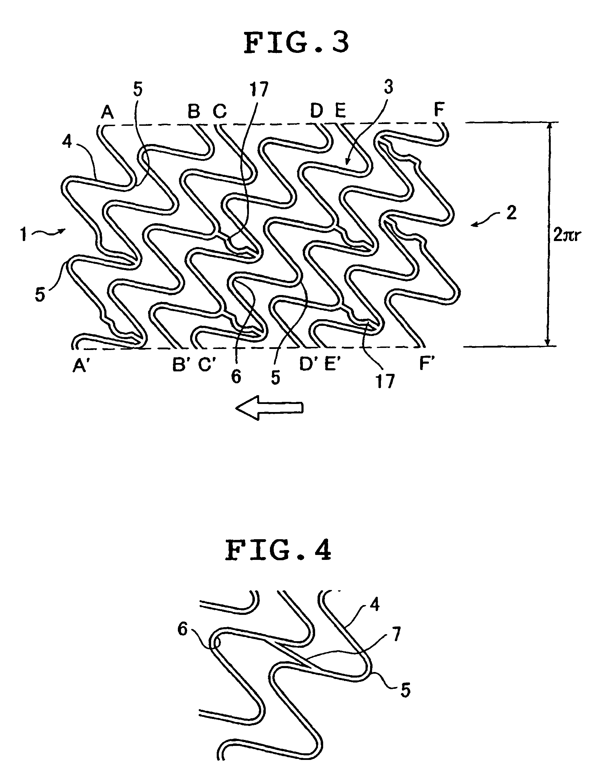

Next, as in the case of connecting members 17 of Fig. 3, the connecting members may be curved or, as in the case of the connecting member 7, they may be straight. If they are curved, they can contribute to the flexibility of the stent as a whole. If they are straight, they can substantially prevent changes in the stent length due to the expansion/contraction of the stent diameter, so that it is desirable for them to be straight at main positions. In the example shown in Fig. 1, adjacent ribbon-like bodies having wave-forms are arranged in a parallel pattern, and apexes of the waves are connected by the thin and narrow connecting member 7. If, instead of connecting the apexes of the waves of the connecting members 7, it is also possible to connect the inflexion points of the waves, as shown in Fig. 4, thereby achieving the same effect. Further, instead of connecting the apexes of the waves, it is also possible to connect the bottoms of the waves (in the opposite direction, the apexes of the waves), thereby achieving the same effect. From the viewpoint of deformation of the protruding portions or non-protruding portions due to expansion/contraction of the stent diameter, the connection of the inflection points as shown in Fig. 4 is desirable.

Further, the cylindrical wall portion of the stent of the present invention can be covered with a cover member. The cover member consists of a film of a biologically inactive or compatible material. It is flexible and does not hinder the expansion/contraction of the stent; it covers the outer surface of the stent, enabling it to smoothly move in the vessel, helping to restore the vessel, imparting biocompatibility, and gradually removing chemicals contained in the film. Examples of the film material include porous polyurethane, teflon, and fluororesin. It is also possible for the film to be formed of a material having biodegradability or bioabsorbability. Also in the case in which the cover member is provided, the stent of the present invention provides the same effect.

In the preparing method of the stent of the present invention, the above-described pattern of thin and narrow members is attached to a cylindrical wall of a stainless steel or the like having distal and proximal ends, and the wall portion other than this pattern is melted by etching technique, such as laser etching or chemical etching, to thereby form an opening. Alternatively, the cylindrical wall allows formation of an opening by cutting according to a pattern by a laser cutting technique on the basis of pattern information stored in a computer. This laser cutting technique is also applicable to a cylindrical wall of plastic. Examples of the material for the cylindrical wall of the stent include a metal plate of silver, tantalum, stainless steel, gold, titanium or the like, a metal such as shape memory alloy, various high molecular plastics, and biodegradable or bioabsorbable plastic.

Next, the way the stent of the present invention is used will be described. The stent of the present invention is fitted onto the cylindrical portion of the balloon of a balloon catheter and secured thereto, and inserted from the base end of a guiding catheter inserted into a vessel in the body, the stent being introduced to a target position of the vessel, e.g., an occluded portion thereof. Next, a fluid is introduced into the balloon under pressure to thereby expand the balloon to a predetermined diameter. At this time, the stent is also expanded. When the stent has been expanded to a predetermined diameter, the fluid in the balloon is extracted to cause it to shrivel. Thereafter, the balloon catheter, the guiding catheter, and the introducer are pulled out to complete the operation of expanding the occluded portion of the vessel.

Next, in the stent of the present invention, when a protruding portion and a non-protruding portion are regarded as one set with a fixed wavelength, only the wavelength increases by the expansion magnification at the time of expansion, and the number of rounds of the spiral remains unchanged, so that even in the case of expansion, the stent length does not decrease as in the case of a conventional spiral stent, the stent length remaining unchanged.

Further, when the stent of the present invention is passed through a curved vessel, it is bent in a smooth curve since the pattern of its thin and narrow members generally constitutes a spiral form, and it is bent in a flexible manner.

Further, in the stent of the present invention, the connecting members consisting of thin and narrow members approximately maintain the pitch of the spiral, so that if the stent is bent in a curved vessel, the stent does not increase in length but maintains a substantially fixed length.

Further, even in a curved vessel, the protruding portion of the stent of the present invention is arcuate, and the outer side of the stent is always rounded, so that the stent does not get caught during operation in the vessel. This will be readily understood from the fact that a straight thin plate can be easily bent both ways, while a curled thin plate is easy to bend in the direction in which it is curled but hard to bend the other way.

Specific embodiments of the present invention will now be described. These embodiments should not be construed restrictively.

(Embodiment 1)

Fig. 1 shows Embodiment 1 of the present invention.

As shown in the development of Fig. 1, the stent of this embodiment has a cylindrical wall portion 3 of stainless steel with a distal end 1 and a proximal end 2. The cylindrical wall portion is formed of thin and narrow members 4 of stainless steel. The thin and narrow members form a ribbon-like body in which wave-like protruding portions 51 and non-protruding portions 6 adjacent thereto are alternately arranged and which is spirally wound around into a cylindrical wall portion. In the example shown in Fig. 1, the wave-like protruding portions 5 of the ribbon-like body and the wave-like non-protruding portions 6 adjacent thereto come near and put into each other to thereby form a wall portion formed of thin and narrow members having high density.

Further, the protruding portions 5 of the first ribbon-like body 21 are connected to the protruding portions 51 of the second ribbon-like body 22 by the connecting member 7 by way of the non-protruding portions 6 of the first ribbon-like bodies 21 adjacent thereto. The connecting position is determined such that the spiral pitch of the ribbon-like bodies does not change whether the stent is expanded or contracted, so that the stent length remains substantially the same. Thus, there is no need to perform length calculation before and after the expansion or contraction of the stent.

(Embodiment 2)

Fig. 5 is a development showing another embodiment of the present invention. This embodiment differs from Embodiment 1 in that while Embodiment 1 basically adopts a wave-shaped continuous pattern, this embodiment basically adopts a zigzag-shaped continuous pattern. The zigzag pattern of Embodiment 2 is in the same state as when a single ribbon-like body is wound. Connection is established between portions A and A', B and B', ... , G and G', and H and H' to form a cylindrical wall portion.

(Embodiment 3)

Another embodiment of the stent of the present invention will be described with reference to Fig. 6. This embodiment differs from Embodiment 1 in that while the basic pattern of Embodiment 1 is a continuous wave-like pattern, this embodiment adopts a pattern in which elliptical frames 13 are formed of thin and narrow members, the portions encircled by the ellipses constituting openings 14. The adjacent intersections 15, 25 of the minor axes of the ellipses and the elliptical frames are connected by bar member 16 formed of thin and narrow members, and through repetition of this connection, a basic pattern is obtained which is as a whole in the form of a rosary-like continuous pattern 18. Otherwise, it is the same as the wave-like continuous basic pattern of Embodiment 1. Here, in the ribbon-like bodies (basic patterns) of Embodiment 3, by connecting adjacent bar members 16 by a connecting member 7 formed of a thin and narrow member, the same effect as that of Embodiment 1 is obtained.

(Embodiment 4)

Another embodiment of the stent of the present invention will be described. This embodiment (not shown) differs from Embodiment 3 only in that while in Embodiment 3 elliptical frame portions are formed of thin and narrow members, in this embodiment, rhombic frame portions are formed of the thin and narrow members. Thus, Embodiment 4 provides the same effect as Embodiment 3.

(Embodiment 5)

Another embodiment of the present invention, which is more flexible than the above embodiments, will be described. This embodiment differs from Embodiment 1 in that while Embodiment 1 adopts linear connecting members, this embodiment uses U-shaped meandering connecting members 17 as shown in Fig. 3. Otherwise, it has the same structure as Embodiment 1. Embodiment 5 provides the same effect as Embodiment 1. Further, it is advantageous in that it is flexible to bending in the major axis line direction of the cylindrical body. Apart from the U-shaped structure, the connecting members may be of a W-shaped, N-shaped, wave-shaped, or zigzag-shaped structure. Further, if the meandering connecting members of Embodiment 5 are used in Embodiments 2 through 4, the effect of flexibility with respect to the bending in the major axis line direction of the cylindrical body can be achieved as in Embodiment 5.

As described above, in the stent of the present invention, the thin and narrow members are formed into a spiral structure composed of ribbon-like bodies in which protruding portions and non-protruding portions are formed continuously, with the protruding portions and the adjacent non-protruding portions thereof getting into each other, whereby a wall portion of high density is formed of the thin and narrow members. Thus, even in the state in which the stent is expanded, the protruding portions and the non-protruding portions solely extend by the expanding magnification, and the number of rounds of the spiral remains the same, so that the stent length is not reduced as in the conventional spiral stent, the stent length being the same before and after the expansion.

Further, in the stent of the present invention, the connecting members connecting the thin and narrow members of adjacent ribbon-like bodies and formed of thin and narrow members are at a crossing angle with respect to the longitudinal direction of the ribbon-like bodies, and the connecting members are formed such that the distance between the central axes of the ribbon-like bodies is substantially the same before and after the deformation of the thin and narrow members as a result of a change in the diameter of the stent, so that the stent length does not increase if the stent is bent in a curved vessel, the stent maintaining a substantially fixed length.

Further, in the stent of the present invention, there is provided at least one connecting member for one round of the ribbon-like body, and the central line of the protruding portions of the thin and narrow members is at a crossing angle with respect to the major axis line parallel to the central axis of the cylindrical body and passing the surface of the cylindrical wall portion. At least two, more preferably three, of the protruding portions of the thin and narrow members are not connected by the connecting members, so that, with the spiral structure of the pattern of the thin and narrow members of the stent, the stent is bent in a smooth curve when passing through a curved vessel, and the bending is effected with flexibility.

Further, the stent of the present invention forms an arcuate body along the curve of the curved surface of the cylindrical wall portion, so that the round back is always directed toward the outside of the stent, whereby it does not get caught during operation in the vessel.

Further, in the stent of the present invention, the central line of the protruding portions of the thin and narrow members is at an angle of 20 to 70 degrees, and more preferably, 30 to 60 degrees, with respect to the major axis line of the cylindrical body parallel to the central axis of the cylindrical wall portion, whereby even in a curved vessel, the protruding portions in the whole form an arcuate body, thereby making it possible to prevent the stent from being caught.

Further, in the stent of the present invention, a thin and narrow member spirally covers the cylindrical wall portion in a form of a ribbon-like body, and the connecting members are arranged at intervals of 60 to 180 degrees of the entire round of 360 degrees along the spiral direction of the ribbon-like body, whereby the stent is bent in a smooth curve when passing through a curved vessel, and the bending is effected with flexibility.

Disclosed is a stent which can be smoothly inserted into a curved body duct with less danger of being caught and can be easily placed. A stent includes a cylindrical wall portion arranged such that a thin and narrow member with a bottom surface of the section extending along the curved surface of the cylindrical wall portion spirally forms of a ribbon-like body, wherein the ribbon-like body includes protruding portions of the thin and narrow member and non-protruding portions thereof, and wherein the stent further includes a connecting member which connects between adjacent ribbon-like body.

**Claims**

1. A stent comprising a cylindrical wall portion (3) with distal and proximal ends (1, 2), the stent being dilatable by a balloon, wherein:

   the cylindrical wall portion (3) is arranged such that thin and narrow members (4) whose cross-sectional form has a bottom surface extending along the curved surface of the cylindrical wall portion spirally form ribbon-like bodies (21, 22);
   the ribbon-like bodies (21, 22) include protruding portions (5) and non-protruding portions (6) thereof, the protruding portions (5) forming a curved surface of the cylindrical wall portion (3); and
   the stent further comprises connecting members (7; 17) formed of thin and narrow members, which connect the thin and narrow members (4) of adjacent ribbon-like bodies to form a cylindrical body and which are provided at least one for the distance of one round of the ribbon-like bodies (21, 22) around the cylindrical body, but at least two of the protruding portions (5) of the ribbon-like bodies (21, 22) are not connected by the connecting members (7; 17),

   **characterized in that**
   central lines (10) of the protruding portions (5) are at an angle with respect to a major axis line parallel to the central axis of the cylindrical body and passing the surface of the cylindrical wall portion (3); and
   the protruding portions (5) form an arcuate body along the cylindrical wall portion (3) and occupy not less than 0.58 times a circumferential length of the ellipsoid obtained by cutting the cylindrical body in a direction (8) perpendicular to the central lines (10) of the protruding portions (5).

2. A stent according to Claim 1, wherein the protruding portions (5) of a first ribbon-like body (21) and the non-protruding portions (6) of a second ribbon-like body (22) adjacent to the first ribbon-like body come near and put into each other in the direction of the central lines (10) of the protruding portions (5) of the first ribbon-like body to thereby form a cylindrical wall portion (3) with high density with the thin and narrow members (4).

3. A stent according to Claim 1 or 2, wherein the protruding portions (5) of the thin and narrow members (4) form the respective ribbon-like body (21, 22) in a continuous wave-like or continuous zigzag-shaped structure.

4. A stent according to Claim 3, wherein the ribbon-like bodies (21, 22) are formed in a continuous wave-like structure, and the connecting members (7) connect the inflexion points of the waves.

5. A stent according to Claim 1 or 2, wherein each protruding portion (5) of the thin and narrow members (4) constitutes a part of a rhombus or ellipsoid (13), adjacent rhombi or ellipsoids (13) being connected with each other in the longitudinal direction of the respective ribbon-like body by a thin and narrow member (16) to form the ribbon-like body as a whole in the form of a rosary (18).

6. A stent according to one of Claims 1 through 5, wherein the connecting members (7; 17) are provided at intervals of 60 to 180 degrees with respect to one round of 360 degrees along the longitudinal direction of the spiral of the respective ribbon-like body (21, 22).

7. A stent according to one of Claims 1 through 6, wherein the connecting members (17) are curved.

8. A stent according to one of Claims 1 through 7, wherein the cylindrical wall portion (3) consists of a metal or plastic cylindrical wall portion with a plurality of openings (14) formed in the wall surface thereof.

9. A stent according to one of Claims 1 through 8, wherein the central lines (10) of the protruding portions (5) of the ribbon-like bodies (21, 22) are at an angle of 20 to 70 degrees with respect to the major axis line parallel to the central axis of the cylindrical body and passing the surface of the wall portion.

**Patentansprüche**

1. Stent mit einem zylindrischen Wandabschnitt (3) mit einem distalen und einem proximalen Ende (1, 2), wobei der Stent durch einen Ballon ausweitbar ist, wobei:

der zylindrische Wandabschnitt (3) derart angeordnet ist, dass dünne und schmale Elemente (4), deren Querschnittsform eine Bodenfläche hat, die sich entlang der gekrümmten Fläche von dem zylindrischen Wandabschnitt erstreckt, spiralartig bandartige Körper (21,22) ausbilden;
die bandartigen Körper (21, 22) von ihnen vorragende Abschnitte (5) und nicht vorragende Abschnitte (6) haben, wobei die vorragenden Abschnitte (5) eine gekrümmte Fläche von dem zylindrischen Wandabschnitt (2) ausbilden; und
der Stent des weiteren Verbindungselemente (7; 17) aufweist, die aus dünnen und schmalen Elementen ausgebildet sind und die die dünnen und schmalen Elemente (4) der benachbarten bandartigen Körper verbinden, um einen zylindrischen Körper auszubilden, und bei denen zumindest einer für den Abstand von einer Umrundung der bandartigen Körper (21, 22) um den zylindrischen Körper vorgesehen ist, aber wobei zumindest zwei der vorragenden Abschnitte (5) von den bandartigen Körpern (21, 22) nicht durch die Verbindungselemente (7; 17) verbunden sind,

**dadurch gekennzeichnet, dass**
Mittellinien (10) von den vorragenden Abschnitten (5) bei einem Winkel in Bezug auf eine Hauptachsenlinie sind, die parallel zu der Mittelachse von dem zylindrischen Körper ist, und die Oberfläche von dem zylindrischen Wandabschnitt (3) passieren; und
wobei die vorragenden Abschnitte (5) einen gebogenen Körper entlang dem zylindrischen Wandabschnitt (3) ausbilden und nicht weniger als das 0,58-fache von einer Umfangslänge von dem Ellipsoid einnehmen, der erhalten wird durch Aufschneiden des zylindrischen Körpers in eine Richtung (8), die senkrecht zu den Mittellinien (10) der vorragenden Abschnitte (5) steht.

2. Stent gemäß Anspruch 1, wobei
die vorragenden Abschnitte (5) von einem ersten bandartigen Körper (21) und die nicht vorragenden Abschnitte (6) von einem zweiten bandartigen Körper (22), der benachbart zu dem ersten bandartigen Körper ist, sich nähern und ineinander gesetzt sind in der Richtung der Mittellinien (10) der vorragenden Abschnitte (5) von dem ersten bandartigen Körper, um **dadurch** einen zylindrischen Wandabschnitt (3) mit einer hohen Dichte mit den dünnen und schmalen Elementen (4) auszubilden.

3. Stent gemäß Anspruch 1 oder 2, wobei
die vorragenden Abschnitte (5) von den dünnen und schmalen Elementen (4) die jeweiligen bandartigen Körper (21, 22) in einem kontinuierlichen wellenartigen oder kontinuierlichen zickzackförmigen Aufbau ausbilden.

4. Stent gemäß Anspruch 3, wobei
die bandartigen Körper (21, 22) in einem kontinuierlichen wellenartigen Aufbau ausgebildet sind und die Verbindungselemente (7) die Wendepunkte der Welle verbinden.

5. Stent gemäß Anspruch 1 oder 2, wobei
jeder vorragende Abschnitt (5) von den dünnen und schmalen Elementen (4) einen Teil von einem Rhombus oder Ellipsoid (13) bildet, wobei benachbarte Rhomben oder Ellipsoide (13) miteinander in der Längsrichtung von dem jeweiligen bandartigen Körper durch ein dünnes und schmales Element (16) verbunden sind, um den bandartigen Körper als Ganzes in der Form eines Rosenkranzes (18) auszubilden.

6. Stent gemäß einem der Ansprüche 1 bis 5, wobei
die Verbindungselemente (7; 17) bei Intervallen von 60 bis 180° in Bezug auf eine Umrundung von 360° entlang der Längsrichtung der Spirale von dem jeweiligen bandartigen Körper (21, 22) vorgesehen sind.

7. Stent gemäß einem der Ansprüche 1 bis 6, wobei die Verbindungselemente (17) gekrümmt sind.

8. Stent gemäß einem der Ansprüche 1 bis 7, wobei der zylindrische Wandabschnitt (3) aus einem metallischen oder aus Kunststoff bestehenden zylindrischen Wandabschnitt mit einer Vielzahl an Öffnungen (14) besteht, die in der Wandfläche von diesem ausgebildet sind.

**9.** Stent gemäß einem der Ansprüche 1 bis 8, wobei die Mittellinien (10) von den vorragenden Abschnitten (5) von den bandartigen Körpern (21, 22) bei einem Winkel von 20 bis 70° in Bezug auf die Hauptachsenlinie sind, die parallel der Mittelachse von dem zylindrischen Körper ist, und die Oberfläche von dem Wandabschnitt durchtreten.

**Revendications**

**1.** Stent comprenant une partie paroi cylindrique (3) ayant des extrémités distale et proximale (1, 2), le stent pouvant être dilaté par un ballonnet, dans lequel :

la partie paroi cylindrique (3) est conçue de telle manière que des éléments minces et étroits (4) dont la forme en section transversale comporte une surface inférieure qui s'étend le long de la surface incurvée de la partie paroi cylindrique forment en spirale des corps en forme de rubans (21, 22) ;
les corps en forme de rubans (21, 22) comportent des parties saillantes (5) et des parties non saillantes (6), les parties saillantes (5) formant une surface incurvée de la partie paroi cylindrique (3) ; et
le stent comprend en outre des éléments de connexion (7 ; 17) constitués d'éléments minces et étroits, qui relient les éléments minces et étroits (4) de corps en forme de rubans voisins pour former un corps cylindrique et qui sont placés au moins un pour la distance d'un tour des corps en forme de rubans (21, 22) autour du corps cylindrique, mais au moins deux des parties saillantes (5) des corps en forme de rubans (21, 22) ne sont pas connectées par les éléments de connexion (7 ; 17),

**caractérisé en ce que** :

les axes (10) des parties saillantes (5) forment un angle par rapport à un axe principal parallèle à l'axe du corps cylindrique et qui passe par la surface de la partie paroi cylindrique (3) ; et
les parties saillantes (5) forment un corps arqué le long de la partie paroi cylindrique (3) et occupent au moins 0,58 fois la longueur périphérique de l'ellipse obtenue en coupant le corps cylindrique dans une direction (8) perpendiculaire aux axes (10) des parties saillantes (5).

**2.** Stent selon la revendication 1, dans lequel les parties saillantes (5) d'un premier corps en forme de ruban (21) et les parties non saillantes (6) d'un deuxième corps en forme de ruban (22) voisin du premier corps en forme de ruban se rapprochent et entrent les unes dans les autres dans la direction des axes (10) des parties saillantes (5) du premier corps en forme de ruban pour former ainsi une partie paroi cylindrique (3) de densité élevée avec les éléments minces et étroits (4).

**3.** Stent selon la revendication 1 ou 2, dans lequel les parties saillantes (5) des éléments minces et étroits (4) mettent le corps en forme de ruban (21, 22) respectif sous la forme d'une structure ondulée continue ou d'une structure en zigzag continue.

**4.** Stent selon la revendication 3, dans lequel les corps en forme de rubans (21, 22) sont mis sous la forme d'une structure ondulée continue, et les éléments de connexion (7) relient les points d'inflexion des ondes.

**5.** Stent selon la revendication 1 ou 2, dans lequel chaque partie saillante (5) des éléments minces et étroits (4) constitue une partie d'un losange ou d'un ellipsoïde (13), les losanges ou ellipsoïdes (13) voisins étant reliés entre eux dans la direction longitudinale du corps en forme de ruban respectif par un élément mince et étroit (16) pour mettre le corps en forme de ruban dans son ensemble sous la forme d'un chapelet (18).

**6.** Stent selon l'une des revendications 1 à 5, dans lequel les éléments de connexion (7 ; 17) sont placés à des intervalles de 60 à 180 degrés par rapport à un tour de 360 degrés suivant la direction longitudinale de la spirale du corps en forme de ruban (21, 22) respectif.

**7.** Stent selon l'une des revendications 1 à 6, dans lequel les éléments de connexion (17) sont incurvés.

**8.** Stent selon l'une des revendications 1 à 7, dans lequel la partie paroi cylindrique (3) est constituée d'une partie paroi cylindrique en métal ou en plastique comportant une pluralité d'ouvertures (14) formées dans sa surface de paroi.

**9.** Stent selon l'une des revendications 1 à 8, dans lequel les axes (10) des parties saillantes (5) des corps en forme de rubans (21, 22) forment un angle de 20 à 70 degrés par rapport à l'axe principal du corps cylindrique et passent par la surface de la partie paroi.

# FIG.1

# FIG.2

EP 1 245 203 B1

# FIG.3

# FIG.4

12

# FIG.5

# FIG.6

# FIG.7

# FIG.8